(19) Europäisches Patentamt
European Patent Office
Office européen des brevets

(11) Veröffentlichungsnummer: **0 176 032**
**B1**

(12) **EUROPÄISCHE PATENTSCHRIFT**

(45) Veröffentlichungstag der Patentschrift: **07.03.90**

(21) Anmeldenummer: **85111835.6**

(22) Anmeldetag: **19.09.85**

(51) Int. Cl.$^5$: **C 07 C 65/38**, C 07 C 65/19, C 07 C 63/66, C 07 C 35/36, C 07 C 47/57, C 07 C 69/76, C 07 C255/49, C 07 C255/53, C 07 C255/56, C 07 C233/64, C 07 C247/02

# (54) Tetralin-Derivate, ihre Herstellung und Verwendung.

(30) Priorität: **22.09.84 DE 3434942**

(43) Veröffentlichungstag der Anmeldung: **02.04.86 Patentblatt 86/14**

(45) Bekanntmachung des Hinweises auf die Patenterteilung: **07.03.90 Patentblatt 90/10**

(84) Benannte Vertragsstaaten: **AT BE CH DE FR GB IT LI NL SE**

(56) Entgegenhaltungen:
**EP-A- 0 002 742**
**EP-A- 0 084 667**
**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Frickel, Fritz-Frieder, Dr.**
**Silvanerweg 7**
**D-6705 Deidesheim (DE)**
Erfinder: **Wuest, Hans-Heiner, Dr.**
**Unteres Bieth 10**
**D-6901 Dossenheim (DE)**
Erfinder: **Nuerrenbach, Axel, Dr.**
**Koenigsberger Strasse 7**
**D-6718 Gruenstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 176 032 B1**

Jouve, 18, rue St-Denis, 75001 Paris, France

**Beschreibung**

Die vorliegende Erfindung betrifft neue Tetralin-Derivate, deren Herstellung sowie deren Verwendung bei der Bekämpfung von Krankheiten.

Es ist beispielsweise aus den DE-OS 28 54 354 und 32 02 118 bekannt, daß Vinylbenzoesäure-Derivate pharmakologische Wirkungen bei der topischen und systemischen Therapie von Neoplasien, Akne, Psoriasis und anderen dermatologischen Affektionen aufweisen. Diese Vinylbenzoesäure-Derivate befriedigen jedoch nicht immer, vermutlich weil bei ihnen — in untenstehender Formel I ausgedrückt — X frei von Sauerstoff ist. Es wurde gefunden, daß Tetralin-Derivate der Formel I

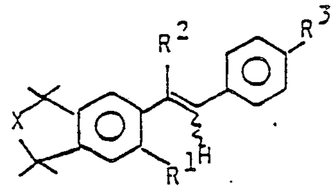

(I)

in der

$R^1$ ein Wasserstoffatom, eine $C_{1-4}$-Alkyl- oder $C_{1-4}$-Alkoxygruppe oder ein Halogenatom,

$R^2$ ein Wasserstoffatom oder eine $C_{1-6}$-Alkyl- oder $C_{3-6}$-Cycloalkylgruppe,

X eine $-CH_2-CO-$, $-CH_2-CHOH-$, $-CH_2-C(R^4)OH-$, $-CH_2-C(OCOR^4)H-$, $-CH_2-C(R^4)(OCHOR^5)-$, $-CHOH-CHOH-$, $-CH(OCOR^4)-CH(OCOR^4)-$, $-CH_2-CH(OR^4)-$ oder

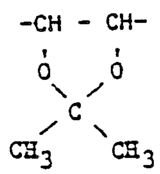

-Gruppe (mit $R^4$ und $R^5$ in der Bedeutung von $C_{1-4}$-Alkyl) und

$R^3$ eine Nitril- oder $C_{2-10}$-Ketalgruppe oder eine $-CH(R^7)(R^8)-$ oder $-CO-R^9$, worin $R^7$ ein Wasserstoffatom oder eine $C_{1-3}$-Alkylgruppe, $R^8$ ein Wasserstoffatom, eine $C_{1-3}$-Alkylgruppe oder einen Rest $-OR^{10}$ oder $-NR^{11}R^{12}$ (mit $R^{10}$ in der Bedeutung von Wasserstoff, $C_{1-4}$-Alkyl, $C_{2-20}$-Alkanoyl oder gegebenenfalls substituiertes Benzoyl und $R^{11}$ und $R^{12}$ in der Bedeutung von Wasserstoff, $C_{1-4}$-Alkyl, $C_{2-20}$-Alkanoyl, gegebenenfalls substituiertem Benzoyl oder $R^{11}$ und $R^{12}$ zusammen mit dem Stickstoffatom, an das sie gebunden sind, in der Bedeutung eines heterocyclischen Rests), und $R^9$ ein Wasserstoff- oder Halogenatom, eine $C_{1-4}$-Alkylgruppe, den Azido-, Imidazol- oder Triazolrest oder die Gruppen $-OR^{13}$ oder $-NR^{14}R^{15}$ bedeutet (mit $R^{13}$ in der Bedeutung von Wasserstoff, $C_{1-8}$-Alkyl, mit einer oder mehreren Hydroxygruppen oder einer $C_{1-4}$-Alkoxygruppe substituiertes $C_{2-6}$-Alkyl, gegebenenfalls substituiertes Aryl, gegebenenfalls im Arylteil substituiertes Aralkyl und $R^{14}$ und $R^{15}$ in der Bedeutung von Wasserstoff, $C_{1-6}$-Alkyl, mit einer oder mehreren Hydroxygruppen substituiertes $C_{2-6}$-Alkyl, einer Aryl- oder Tetrazoylgruppe oder $R^{14}$ und $R^{15}$ zusammen mit dem Stickstoffatom, an das sie gebunden sind, in der Bedeutung eines heterocyclischen Ringsystems mit 3-6 Ringatomen) bedeuten, sowie gegebenenfalls deren physiologisch verträglichen Salze, ein besseres Wirkungsspektrum besitzen.

Bevorzugt sind die all-E-Verbindungen und von diesen wiederum besonders diejenigen, in denen X die Gruppen $-CH_2-CO-$, $-CH_2-CHOH-$, oder $-CHOH-CHOH$, $R^1$ Wasserstoff, Methyl, Methoxy oder Fluor, $R^2$ Wasserstoff oder Methyl und $R^3$ die Gruppen $-COOH$, $-COO-C_{1-4}$-alkyl, $-CHO$, $-CH_2OH$ oder $CH_2-O-CO-CH_3$ bedeuten.

Typische Beispiele für erfindungsgemäße Verbindungen sind die folgenden Tetralinverbindungen, die in der para-Position des Phenylringes noch mit den weiter unten aufgeführten Resten ($R^3$ der allgemeinen Formel I) substituiert sind.

1,2,3,4-Tetrahydro-1,1,4,4-tetramethyl-3-oxo-6-(1-methyl-2-phenylethenyl)-naphthalin
1,2,3,4-Tetrahydro-1,1,4,4-tetramethyl-2-oxo-6-(1-methyl-2-phenylethenyl)-naphthalin
1,2,3,4-Tetrahydro-1,1,4,4-tetramethyl-3-hydroxy-3-methyl-6-(1-methyl-2-phenylenethenyl)naphthalin
1,2,3,4-Tetrahydro-1,1,4,4-tetramethyl-3-hydroxy-3-ethyl-6-(1-methyl-2-phenylethenyl)naphthalin
1,2,3,4-Tetrahydro-1,1,4,4-tetramethyl-3-hydroxy-3-propyl-6-(1-methyl-2-phenylethenyl)naphthalin
1,2,3,4-Tetrahydro-1,1,4,4-tetramethyl-3-hydroxy-3-butyl-6-(1-methyl-2-phenylethenyl)naphthalin
1,2,3,4-Tetrahydro-1,1,4,4-tetramethyl-2-methoxy-6-(1-methyl-2-phenylethyl)naphthalin
1,2,3,4-Tetrahydro-1,1,4,4-tetramethyl-3-methoxy-6-(1-methyl-2-phenylethenyl)naphthalin
1,2,3,4-Tetrahydro-1,1,4,4-tetramethyl-2-ethoxy-6-(1-methyl-2-phenylethenyl)naphthalin
1,2,3,4-Tetrahydro-1,1,4,4-tetramethyl-3-ethoxy-6-(1-methyl-2-phenylethenyl)naphthalin
1,2,3,4-Tetrahydro-1,1,4,4-tetramethyl-3-propoxy-6-(1-methyl-2-phenylethenyl)naphthalin

1,2,3,4-Tetrahydro-1,1,4,4-tetramethyl-2-propoxy-6-(1-methyl-2-phenylethenyl)naphthalin
1,2,3,4-Tetrahydro-1,1,4,4-tetramethyl-3-butoxy-6-(1-methyl-2-phenylethenyl)naphthalin
1,2,3,4-Tetrahydro-1,1,4,4-tetramethyl-2-butoxy-6-(1-methyl-2-phenyl-ethenyl)naphthalin
1,2,3,4-Tetrahydro-1,1,4,4-tetramethyl-2-acetoxy-6-(1-methyl-2-phenylethenyl)naphthalin
1,2,3,4-Tetrahydro-1,1,4,4-tetramethyl-3-acetoxy-6-(1-methyl-2-phenylethenyl)naphthalin
1,2,3,4-Tetrahydro-1,1,4,4-tetramethyl-2-propanoyloxy-6-(1-methyl-2-phenylethenyl)naphthalin
1,2,3,4-Tetrahydro-1,1,4,4-tetramethyl-3-propanoyloxy-6-(1-methyl-2-phenylethenyl)naphthalin
1,2,3,4-Tetrahydro-1,1,4,4-tetramethyl-2-butanoyloxy-6-(1-methyl-2-phenylethenyl)naphthalin
1,2,3,4-Tetrahydro-1,1,4,4-tetramethyl-3-butanoyloxy-6-(1-methyl-2-phenylethenyl)naphthalin
1,2,3,4-Tetrahydro-1,1,4,4-tetramethyl-2-pentamoyloxy-6-(1-methyl-2-phenylethenyl)naphthalin
1,2,3,4-Tetrahydro-1,1,4,4-tetramethyl-3-pentanoyloxy-6-(1-methyl-2-phenylethenyl)naphthalin
1,2,3,4-Tetrahydro-1,1,4,4-tetramethyl-2-hydroxy-6-(1-methyl-2-phenylethenyl)naphthalin
1,2,3,4-Tetrahydro-1,1,4,4-tetramethyl-3-hydroxy-6-(1-methyl-2-phenylethenyl)naphthalin
1,2,3,4-Tetrahydro-1,14,4-tetramethyl-2,3-dihydroxy-6-(1-methyl-2-phenylethenyl)naphthalin
1,2,3,4-Tetrahydro-1,1,4,4-tetramethyl-2-hydroxy-2-methyl-6-(1-methyl-2-phenylethenyl)naphthalin
1,2,3,4-Tetrahydro-1,1,4,4-tetramethyl-2-hydroxy-2-ethyl-6-(1-methyl-2-phenylethenyl)naphthalin
1,2,3,4-Tetrahydro-1,1,4,4-tetramethyl-2-hydroxy-2-propyl-6-(1-methyl-2-phenylethenyl)naphthalin
1,2,3,4-Tetrahydro-1,1,4,4-tetramethyl-2-hydroxy-2-butyl-6-(1-methyl-2-phenylethenyl)naphthalin
1,2,3,4-Tetrahydro-1,1,4,4-tetramethyl-3-hydroxy-3-methyl-6-(1-methyl-2-phenylethenyl)naphthalin
1,2,3,4-Tetrahydro-1,1,4,4-tetramethyl-2,3-diacetoxy-6-(1-methyl-2-phenylethenyl)naphthalin
1,2,3,4-Tetrahydro-1,1,4,4-tetramethyl-2,3-dipropanoyloxy-6-(1-methyl-2-phenylethenyl)naphthalin
2,2,4,4,9,9-Hexamethyl-6-(1-methyl-2-phenylethyl)-3a,4,9,9a-tetrahydronaphtho [2,3d]-1,3-dioxol

Die voranstehenden Verbindungen enthalten als Rest $R^2$ eine Methylgruppe eingesetzt. Weitere erfindungsgemäße Verbindungen enthalten für den Rest $R^2$ Wasserstoff, Ethyl, Propyl, Cyclopropyl, Butyl, 3-Methylpropyl, Pentyl oder Hexyl.

Diese Verbindungen sind in der para-Position des Phenylringes noch mit den folgenden typischen Resten ($R^3$ der allgemeinen Formel I) substituiert:

Carboxy-, Methoxycarbonyl-, Ethoxycarbonyl-, Propyloxycarbonyl-, (1-Methyl)ethoxycarbonyl-, Butoxycarbonyl-, Octyloxycarbonyl-, (2-Methoxy)-ethoxycarbonyl-, (2-Hydroxy)-ethoxycarbonyl-, (2,3-Dihydroxy)propoxycarbonyl-, Benzyloxycarbonyl-, Cyano-, Formyl-, Hydroxymethyl-, Methyl-, Ethyl-, Propyl-, Chlorformyl-, Fluorformyl-, Azidoformyl-, Methoxymethyl-, Ethoxymethyl-, Propyloxymethyl-, Butyloxymethyl-, Benzyloxymethyl-; Acetoxymethyl-, Propionyloxymethyl-, Hexadecanoyloxymethyl-, Aminomethyl-, Benzoyloxymethyl-, (3,4-Dimethoxy)benzoyloxymethyl-, Methylaminomethyl-, Ethylaminomethyl-, Butylaminomethyl-, Propylaminomethyl-, Acetyl-, Benzoylaminomethyl-, (4-Methoxy)benzoylaminomethyl-, Acetylaminomethyl-, Propionylaminomethyl-, Palmitoylaminomethyl-, Carbamoyl-, (N-Methylcarbamoyl-, [N-(2,3-Dihydroxypropyl)]-carbamoyl-, (N-Ethyl)-carbamoyl-, (N-Hexyl)-carbamoyl-, (N,N'-Dimethyl)-carbamoyl-, Morpholino-carbamoyl-, 3-N-Imidazolyl)-carbonyl-, 3-(N-Triazolyl)-carbonyl-.

Die neuen Verbindungen lassen sich herstellen, indem man eine Carbonylverbindung der Formel (II)

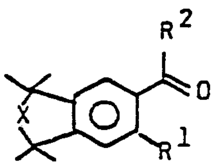

(II)

in der X, $R^1$ und $R^2$ die oben angegebenen Bedeutungen haben mit einer Phosphorverbindung der Formel (III)

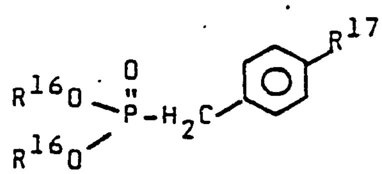

(III)

in der $R^{16}$ eine $C_{1-3}$-Alkylgruppe und $R^{17}$ eine $C_{1-2}$-Alkylgruppe, eine Carbo-$C_{1-8}$-alkoxygruppe bedeutet oder für eine ($C_{1-4}$-Alkoxy) methylgruppe steht, nach Wittig-Horner umsetzt.

Die Umsetzung nach Wittig-Horner verläuft bei einer Temperatur von bis zu 100 °C, zweckmäßig bei 20 bis 50 °C. Die Umsetzung kann bei atmosphärischem Druck oder in einem geschlossenen Gefäß unter erhöhtem Druck, gegebenenfalls unter Erwärmung auf den angegebenen Temperaturbereich durchgeführt werden.

Man kann diese Umsetzung in Gegenwart eines Verdünnungs- oder Lösungsmittels, beispielsweise eines niederen gesättigten Dialkylether, Dialkylglykolethers oder cyclischen Ethers, wie Diethylether,

Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder in Mischungen der genannten Lösungsmittel durchführen. Bevorzugt verwendet man cyclische Ether, wie Dioxan oder Tetrahydrofuran sowie insbesondere Dimethylformamid oder deren Mischungen, wobei die Umsetzung im allgemeinen bei einer Temperatur bis zu 30 °C abläuft.

Die Umsetzungen werden in Gegenwart eines Deprotonierungsmittels für das Phosphat (III) vorgenommen. Geeignet sind Alkalimetallhydride und Alkalimetallamide, insbesondere des Natriums und Kaliums, die Natrium- und Kaliumsalze von Dimethylsulfoxid, Alkyllithiumverbindungen wie n-Butyllithium oder Alkalimetallalkoholate, vorzugsweise Natriummethanolat und Natriumethanolat.

Die Säureester der Formel (I), in denen $R^3$ eine Carboalkoxygruppe bedeutet, werden, falls dies erwünscht ist, in die freien Carbonsäuren durch Esterverseifung überführt. Umgekehrt läßt sich natürlich die freie Säure in bekannter Weise verestern.

Die Hydrolyse läuft in üblicher Weise bei im allgemeinen bis zu 120 °C ab und wird vorzugsweise bei Raumtemperatur durchgeführt.

Sie kann bei atmosphärischem Druck oder in einem geschlossenen Gefäß unter erhöhtem Druck durchgeführt werden.

Zweckmäßigerweise wird die Verseifung/Veresterung in Gegenwart eines Verdünnungs- oder Lösungsmittels, beispielsweise eines Dialkylglykolethers oder cyclischen Ethers, wie 1,2-Dimethoxyethan, Tetrahydrofuran oder Dioxan, eines niederen aliphatischen Ketons, wie Aceton, Methylethylketon oder Methylisobutylketon, die Veresterung insbesondere in dem zur Veresterung vorgesehenen Alkohol, wie Methanol, Ethanol, Propanol oder Isopropanol, gegebenenfalls in Gegenwart von Wasser bzw. in Mischungen der genannten Lösungsmittel mit Wasser durchgeführt.

Bevorzugte Lösungsmittel sind wäßrige Mischungen von Ethanol und Methanol, wobei die Umsetzung beim Siedepunkt des Reaktionsgemisches durchgeführt wird.

Die Verseifung geschieht bevorzugt in Gegenwart von Alkali, wie Alkalimetallhydroxiden, Alkalimetallcarbonaten und -hydrogencarbonaten, insbesondere des Natriums und Kaliums, organischen tertiären Basen, wie Pyridin oder niederen Trialkylaminen, wie Trimethyl- oder Triethylamin in Gemischen mit Wasser. Dabei wird die verwendete Base im Verhältnis zum Ester in stöchiometrischer Menge oder in geringem Überschuß eingesetzt. Vorzugsweise wird Natrium- oder Kaliumhydroxid verwendet.

Die Veresterung geschieht vorteilhaft durch Einleiten von Chlorwasserstoffgas in das Reaktionsgemisch. Die Methylester können auch durch Einwirkung von Diazomethan auf die freie Säure erhalten werden.

Carbonsäurederivate der allgemeinen Formel (I), in der $R^3$ für COOH steht, kann man in ein reaktionsfähiges Säure-Derivat der Formel (IV) überführen

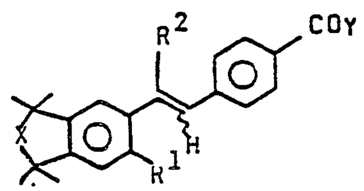

(IV)

in der X, $R^1$ und $R^2$ die oben angegebene Bedeutung haben und Y für einen üblichen, reaktionsfähigen Rest eines gemischten Säureanhydrids oder für ein Halogenatom steht. Diese werden zweckmäßigerweise in einem Lösungsmittel und gegebenenfalls in Gegenwart eines säurebindenden Mittels mit Aminen

$$HN<^{R^{14}}_{R^{15}}$$

oder Alkoholen $HOR^{13}$ zu Amiden und Estern umgesetzt. Die Bezeichnung Y in der Formel (IV) stellt ein Halogenatom, insbesondere Chlor oder auch Brom oder z. B. den N-Oxysuccinimid-Rest dar. Diese Umsetzungen von IV verlaufen bei einer Temperatur von bis zu 50 °C bei Atmosphärendruck oder in einem geschlossenen Gefäß unter erhöhtem Druck.

Man kann diese Umsetzungen in Gegenwart eines Verdünnungs- oder Lösungsmittels, beispielsweise eines niederen gesättigten Dialkylethers, Dialkylglykolethers oder cyclischen Ethers, wie Diethylether, Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder Diethylformamid, oder in Mischungen der genannten Lösungsmittel durchführen. Bevorzugt verwendet man lineare oder cyclische Ether, wie Diethylether oder Tetrahydrofuran sowie insbesondere Dimethylformamid, wobei die Umsetzung im allgemeinen bei einer Temperatur bis zu 30 °C abläuft.

Üblicherweise wird die Umsetzung in Gegenwart einer Base als säurebindendem Mittel vorgenommen. Geeignete Basen sind Alkalimetallcarbonate, -hydrogencarbonate, insbesondere des Natriums und Kaliums, organische tertiäre Basen, wie Pyridin oder niedere Trialkylamine, wie Trimethyl- oder Triethylamin. Dabei wird die verwendete Base im Verhältnis zum eingesetzten Benzoesäurehalogenid in stöchiometrischer Menge oder in geringem Überschuß verwendet.

Eine andere Möglichkeit zur Herstellung der erfindungsgemäßen Verbindungen geht von entsprechenden Säuren der Formel IV aus, wobei in diesem Fall Y für OH steht ; man setzt in Gegenwart eines die Carboxylgruppe aktivierenden, wasserabspaltenden Mittels in einem Lösungsmittel mit Aminen

$$HN{<}^{R^{14}}_{R^{15}}$$ um.

Als wasserabspaltende aktivierende Reagentien können die bei der Peptidsynthese üblichen Reagentien verwendet werden, wie sie beispielsweise in «The Peptides», Band I, Academic Press, N.Y., 1965, Seiten 77 bis 128, beschrieben werden. Das allgemeine Prinzip der Umsetzung besteht in der Aktivierung der Carboxylgruppe, beispielsweise durch Behandlung mit einem Carbodiimid, wie N,N'-Dicyclohexylcarbodiimid, oder durch intermediäre Bildung des Säureazids, eines gemischten Anhydrids (beispielsweise mit Kohlensäuremonoestern), eines aktivierten Esters (beispielsweise des p-Nitrophenylesters) oder eines heterocyclischen Amids (beispielsweise eines Imidazolids) der entsprechenden Carbonsäure (IV).

Die Behandlung einer an der Carboxylgruppe aktivierten Verbindung mit Aminen

$$HN{<}^{R^{14}}_{R^{15}}$$

führt dann zu den erfindungsgemäßen Verbindungen. Die Aktivierungs- und Verknüpfungsreaktionen können in Lösungsmitteln, vorzugsweise in N,N-Dimethylformamid, Tetrahydrofuran, Dioxan, Methylenchlorid, Nitromethan, Acetonitril, Dimethylsulfoxid, N,N-Dimethylacetamid und Hexamethylphosphorsäuretriamid vorgenommen werden. Eine geeignete Temperatur für beide Stufen, d. h., der Reaktion der Säure mit dem Kupplungsmittel und die Reaktion des aktivierten Zwischenproduktes mit Aminen

$$HN{<}^{R^{14}}_{R^{15}}$$

liegt zwischen 20 und 100 °C. Man kann dabei entweder stufenweise vorgehen, indem man das aktivierte Zwischenprodukt vor der Zugabe des Amins isoliert und vorteilhaft, indem man die Reaktionspartner nacheinander ohne Isolierung von Zwischenstufen zur Reaktion bringt. Bei einer bevorzugten Verknüpfungsmethode verwendet man das N,N-Carbonyldiimidazol und arbeitet in Dimethylformamid, wobei die Reaktionstemperatur für beide Stufen bei 20 bis 60 °C liegt.

Zur Herstellung von erfindungsgemäßen Amiden, in denen $R^{15}$ für Wasserstoff steht, ist die direkte Aminolyse von Estern (mit dem Rest $—OR^{13}$) mit primären Aminen $H_2N—R^{15}$ bei Raumtemperatur ohne Lösungsmittel oder gegebenenfalls in Gegenwart eines organischen Lösungsmittels, wir Alkoholen, Ethern, Dialkylformamiden oder Mischungen dieser Lösungsmittel bevorzugt.

Ein Carbonsäurehalogenid der Formel (IV), vorzugsweise das Säurechlorid, kann durch Reaktion mit 2-Aminoethanol oder 2-Amino-2-methylpropanol-1 und anschließende Cyclisierung in ein Oxazolinderivat der Formel (I) überführt werden.

Eine Carbonsäure, ein Carbonsäureester oder ein Carbonsäureamid der Formel (I) kann in an sich bekannter Weise zu den Alkoholen bzw. Aminen der Formel (I) reduziert werden. Vorteilhaft wird die Reduktion mit Hilfe eines Metallhydrids oder Alkalimetallhydrids in Gegenwart eines geeigneten Lösungsmittels durchgeführt. Als Metallhydride werden vorzugsweise komplexe Metallhydride wie Lithiumaluminiumhydrid oder Diisobutylaluminiumhydrid eingesetzt. Als Lösungsmittel werden beim Arbeiten mit Lithiumaluminiumhydrid Ether eingesetzt, wie Diethylether, Dioxan oder Tetrahydrofuran. Führt man die Reduktion aber mit Diisobutylaluminiumhydrid oder einem Alkoxynatriumaluminiumhydrid durch, so ist die Verwendung von Kohlenwasserstoffen wie Hexan oder Toluol bevorzugt.

Ein Amin oder ein Alkohol der Formel (I) kann in an sich bekannter Weise mit einem Alkanoylhalogenid oder -anhydrid, einem Aralkylhalogenid oder -anhydrid oder einem Aroyl- oder Heteroaroylhalogenid oder -anhydrid zweckmäßigerweise in einem inerten Verdünnungs- oder Lösungsmittel, z. B. einem niederen aliphatischen Keton wie Aceton, Methylethylketon oder Methylisobutylketon, einem Dialkylformamid oder mit überschüssigem Acylierungsmittel als Verdünnungs- oder Lösungsmittel in die erfindungsgemäßen Amide und Ester der Formel (I) überführt werden. Bevorzugt werden die Umsetzungen in Gegenwart einer Base als säurebindendes Mittel in einem zwischen — 20 °C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder

niedere Trialkylamine, wie Trimethyl- oder Triethylamin. Dabei können die Basen im Verhältnis zum eingesetzten Alkylierungsmittel in katalytischer Menge oder in stöchiometrischer Menge bzw. in geringem Überschuß verwendet werden.

Ein Alkohol der Formel (I) kann mit Alkylhalogeniden $R^{10}$—J, $R^{10}$—Br oder $R^{10}$—Cl in Gegenwart von Alkalimetallhydriden, vorzugsweise Natriumhydrid oder in Gegenwart von Alkyllithium-Verbindungen, vorzugsweise n-Butyl-lithium, in einem organischen Lösungsmittel wie Tetrahydrofuran, Dioxan, 1,2-Dimethoxyethan, Methyl-tert.-butylether oder bei Verwendung von Natriumhydrid auch in Dimethylformamid in einem zwischen — 10 °C und 40 °C liegenden Temperaturbereich zu einem Ether der Formel (I) umgesetzt werden.

Ein Alkohol der Formel (I) kann mit geeigneten Oxidationsmitteln, vorzugsweise Mangan(IV)-oxid, gegebenenfalls Mangan(IV)-oxid auf einem anorganischen Trägermaterial wie Silicagel oder Aluminiumoxid zu einem Aldehyd der Formel (I) oxidiert werden. Vorteilhaft arbeitet man in einem inerten organischen Lösungsmittel, beispielsweise einem Kohlenwasserstoff wie Hexan oder in einem Ether wie beispielsweise Tetrahydrofuran oder in Mischungen der genannten Lösung- und Verdünnungsmittel im Temperaturbereich zwischen — 10 °C und 30 °C. Die benötigte Reaktionszeit ist im wesentlichen von der Oxidationsaktivität des eingesetzten Mangan-(IV)-oxids abhängig.

Einen Aldehyd der Formel (I) kann man durch Reduktion eines Nitrils der Formel (I) mit Diisobutylaluminiumhydrid in einem Lösungsmittel vorzugsweise in Toluol, Hexan, Tetrahydrofuran oder Mischungen dieser Lösungsmittel in einem Temperaturbereich zwischen — 40 °C und Raumtemperatur erhalten.

Die als Ausgangsmaterialien verwendeten Carbonylverbindungen der allgemeinen Formel (II) können auf verschiedenen Wegen hergestellt werden, die im Prinzip jeweils bekannt sind. Vorzugsweise werden die Grundkörper der allgemeinen Formel (V)

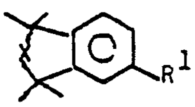

(V)

in der X und $R^1$ die angegebene Bedeutungen haben, auf klassische Weise in die Carbonylverbindungen, vorzugsweise durch Friedel-Crafts-Reaktionen, überführt.

Die erfindungsgemäßen Verbindungen der Formel (I) können einheitlich die cis- oder trans-Struktur haben oder Mischungen der beiden E-/Z-Isomere darstellen. Eine Isomerisierung bei den vorerwähnten Umsetzungen kann außerdem nicht ausgeschlossen werden. Die in solchen Fällen z. B. resultierende Mischung der erfindungsgemäßen Verbindungen der Formel (I) kann durch HPLC-Analyse oder durch ein $^{13}C$-NMR-Spektrum quantitativ bestimmt und das jeweils gewünschte Isomere gegebenenfalls durch fraktionierende Kristallisation oder Chromatographie mit beispielsweise Kieselgelsäulen oder durch präparative HPL-Chromatographie isomerenrein isoliert werden.

Einige der erfindungsgemäßen Verbindungen weisen ein acides Wasserstoffatom auf und können daher mit Basen in üblicher Weise in ein physiologisch verträgliches, gut wasserlösliches Salz überführt werden. Geeignete Salze sind beispielsweise Ammonium-, Alkalimetall-, insbesondere des Natriums, Kaliums und Lithiums, Erdalkalimetallsalze, insbesondere des Calciums oder Magnesiums sowie Salze mit geeigneten organischen Basen, wie mit niederen Alkylaminen, z. B. Methylamin oder Ethylamin, mit substituierten niederen Alkylaminen, insbesondere hydroxysubstituierten Alkylaminen, wie Diethanolamin, Triethanolamin oder Tris-(hydroxymethyl)-aminomethan, Piperidin oder Morpholin.

Erfindungsgemäß erhaltene Amine der Formel (I) können nach bekannter Verfahrensweise in ihre Säureadditionssalze mit physiologisch verträglichen Säuren überführt werden. Als übliche physiologisch verträgliche organische oder anorganische Säure kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzneimittelforschung Band 10, Seiten 224 bis 225, Birkhäuser Verlag, Basel und Stuttgart, 1966, entnommen werden.

Die erfindungsgemäßen Verbindungen und ihre physiologisch verträglichen Salze können aufgrund ihrer pharmakologischen Eigenschaften bei der topischen und systemischen Therapie und auch Prophylaxe von Praekanzerosen und Karzinomen der Haut, der Schleimhäute und innerer Organe sowie bei der topischen und systemischen Therapie von Akne, Psoriasis und anderer mit pathologisch veränderter Verhornung einhergehenden dermatologischen Erkrankungen sowie zur Behandlung von rheumatischen Erkrankungen, insbesondere solcher entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungen die prophylaktische und therapeutische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertreachealgewebe in vitro die nach Vitamin-A-Mangel einsetzende Keratinisierung auf. Diese Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res. 36, 964-972 (1976) oder aus Nature 250, 64-66 (1974) und Nature 253, 47-50 (1975) entnommen werden.

Däruber hinaus werden durch die erfindungsgemäßen Verbindungen die Proliferationsraten bestimmter maligne veränderter Zellen inhibiert. Diese Methodik kann aus J. Natl. Cancer Inst. 60, 1035-1041 (1978), Experimental Cell Research 117, 15-22 (1978) und Proc. Natl. Acad. Sci. USA 77, 2936-2940 (1980) entnommen werden.

Die antiarthritische Wirkung der erfindungsgemäßen Verbindungen kann in üblicher Weise im Tierexperiment im Adjuvans-Arthritis-Modell bestimmt werden. Die dermatologische Aktivität, beispielsweise für die Behandlung von Akne, kan u. a. durch die komedolytische Aktivität und die Fähigkeit nachgewiesen werden, die Anzahl der Zysten im Modell der Rhino-Maus zu reduzieren.

Diese Methode ist von L. H. Kligman et al in The Journal of Investigative Dermatology 73, 354-358 (1979) und von J. A. Mezick et al. in « Models of Dermatology » (Ed. Maibach, Lowe), Vol. 2, S. 59-63, Karger, Basel 1985) beschrieben.

Die Testsubstanz wurde in einem geeigneten Vehikel auf der gesamten Rückenpartie der Rhino-Maus topisch aufgetragen (100 µl), einmal täglich, an fünf aufeinanderfolgenden Tagen pro Woche, zwei Wochen lang ungefähr 72 h nach der letzten Behandlung wurde die dorsale Haut entfernt und in 0,5 %iger Essigsäure 18 h bei 4-5 °C belassen. Danach wurde eine ca. $2 \times 5$ $cm^2$ große Fläche herausgeschnitten, die Epidermis abgeschält, auf einen objektträger aufgebracht (mit der dermalen Seite nach oben) und mittels Alkohol/Xylol wasserfrei gespült, bis die Epidermis durchsichtig erscheint. Die Probe wurde durch überziehen mit Permount fixiert und mikroskopisch ausgewertet. Gemessen wurde der Durchmesser von jeweils 10 Utriculi in jeweils 5 frei gewählten Feldern und daraus durch Vergleich mit der unbehandelten Kontrollgruppe die durchschnittliche Reduktion des Utriculusdurchmessers berechnet. Die folgende Tabelle zeigt die erhaltenen Ergebnisse.

Tabelle

| Substanz | Dosis mg/ml | Reduktion des Utriculus-Durchmessers |
|---|---|---|
| Beispiel 3 | 0,2 | 76,5 |
| | 0,02 | 65,4 |
| Beispiel 5 | 0,02 | 52,6 |
| Beispiel 9 | 0,01 | 52,5 |

Dementsprechend sind ein weiterer Gegenstand der Erfindung therapeutische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines Arzneimittels.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutisch-technischen Hilfsstoffen, entsprechend der gewünschten Applikationsart und mit einer zur Anwendung geeigneten Dosierung, in üblicher Weise beispielsweise durch Vermischen des Wirkstoffs mit den an sich in solchen Präparaten üblichen festen oder flüssigen Träger- und Hilfsstoffen.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,0001 bis 1 %iger Konzentration, bevorzugt in 0,0001 bis 0,1 %iger Konzentration und bei systemischer Anwendung vorzugsweise in einer Einzeldosis von 0,1 bis 50 mg enthalten und täglich in einer oder mehreren Dosierungen je nach Art und Schwere der Erkrankungen verabreicht werden.

Üblicherweise verwendete pharmazeutische technische Hilfsstoffe sind beispielsweise für die lokale Anwendung Alkohole, wie Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol 400, Polyethylenglykol 400-Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxytoluol oder geschmacksverbessernde Zusätze, Stabilisierungsmittel, Emulgiermittel, Gleimittel usw. zugesetzt werden. Voraussetzung ist, daß alle bei der Herstellung pharmazeutischer Zubereitung verwendeten Stoffe toxikologisch unbedenklich sind und mit den verwendeten Wirkstoffen verträglich sind.

Die folgenden Beispiele erläutern die Erfindung

A. Herstellung der Ausgangsmaterialien

6-Acetyl-1,1,4,4-tetramethyl-2-tetralon und 6-Acetyl-1,1,4,4-tetramethyl-3-tetralon

Zu einer Lösung von 47 ml Acetylchlorid in 200 ml Methylenchlorid gibt man portionsweise 117,6 g Aluminium (III) chlorid. Innerhalb 1 h wird eine Lösung von 80 g 1,1,4,4-Tetramethyl-2-tetralon in 120 ml Methylenchlorid zugetropft. Anschließend wird über Nacht bei Raumtemperatur nachgerührt, anderntags wird der Ansatz auf 300 ml Eiswasser gegossen und dreimal mit Methylenchlorid extrahiert. Die organischen Phasen werden über Natriumsulfat getrocknet. Der nach dem Eindampfen der Lösung verbleibende Rückstand wird destilliert. Die zwischen 104 und 125 °C (0,2 bar) siedende Fraktion ergibt 80 g eines 1 : 1-Gemisches der beiden Titelverbindungen.

B. Herstellung der erfindungsgemäßen Verbindungen

Beispiel 1

(E(-4-[2-(5,6,7,8-Tetrahydro-5,5,8,8-tetramethyl-7-oxo-2-naphthalenyl)-1-propenyl]benzoesäureethylester

Zu einer Suspension von 480 ml Dimethylsulfoxid und 18 g 80 %iges Natriumhydrid, das zuvor mit Petrolether vom 20 %igen Paraffin-Anteil befreit worden war, wird innerhalb 1 h eine Lösung von 180 g p-Carboxyethylbenzylphosphonsäurediethylester in 120 ml DMSO bei ca. 35 °C zugetropft.

Anschließend wird noch 45 min bei Raumtemperatur gerührt und innerhalb von 25 min eine Lösung von 73,2 g des Ketongemisches 6-Acetyl-1,1,4,4-tetramethyl-2,3-tetralon in 180 ml Tetrahydrofuran zugetropft.

Am folgenden Tag wird der Ansatz auf 2 l Eiswasser gegossen und mit verdünnter Salzsäure angesäuert. Der entstandene Feststoff wird abfiltriert, (er enthält ein Gemisch des 6-Oxo- und 7-Oxobenzoesäureethylesters) auf dem Filter mit Wasser und und Ethanol gewaschen und aus Essigsäureethylester umkristallisiert. Das Kristallisat wird in 160 ml Methylenchlorid aufgenommen und mit 1,6 Liter n-Heptan versetzt. Der sich bildende Niederschlag wird abfiltriert. Es verbleiben nach dem Trocknen 27,1 g der Titelverbindung, Schmp. 173-174 °C.

Beispiel 2

(E)-4-[2-(5,6,7,8-Tetrahydro-5,5,8,8-tetramethyl-7-oxo-2-naphthalenyl)-1-propenyl]benzoesäure

2 g (E)-4-[2-(5,6,7,8-Tetrahydro-5,5,8,8-tetramethyl-7-oxo-2-naphthalenyl)-1-propenyl]benzoesäureethylester werden mit 0,7 g Kaliumhydroxid in einem Gemisch aus 25 ml Ethanol und 1,5 ml Wasser 3 h bei 80 °C gerührt. Nach dem Überführen der gesamten Reaktionsmasse in 100 ml Wasser wird mit 2 N Salzsäure angesäuert, der erhaltene Niederschlag abfiltriert und mit kaltem Methanol gewaschen. Nach dem Trocknen verbleiben 1,3 g der Titelverbindung, Schmp. 260-261 °C.

Beispiel 3

(E)-4-[2-(5,6,7,8-Tetrahydro-5,5,8,8-tetramethyl-7-hydroxy-2-naphthalenyl)1-propenyl]benzylalkohol

Zu einer gerührten Suspension von 1 g Lithiumaluminiumhydrid in 330 ml Diethylether tropft man eine Lösung von 8,8 g (E)-4-[2-(5,6,7,8-Tetrahydro-5,5,8,8-tetramethyl-7-oxo-2-naphthalenyl)-1-propenyl]benzoesäureethylester in 200 ml Tetrahydrofuran. Nach 4 h bei Rückflußtemperatur versetzt man tropfenweise mit einer gesättigten Weinsäurelösung und extrahiert mehrmals mit Diethylether. Die vereinigten organischen Phasen werden neutral gewaschen, über Natriumsulfat getrocknet und vom Lösungsmittel befreit. Der verbleibende, feste Rückstand wird bei ca. 80 °C in 10 ml Tetrahydrofuran und 100 ml Methanol gelöst und mit 400 ml Wasser versetzt. Das entstehende Kristallisat wird abfiltriert und getrocknet. Es verbleiben 4,7 g der Titelverbindung, Schmp. 184-185 °C.

Beispiel 4

a) (E)-4-[2-(5,6,7,8-Tetrahydro-5,5,8,8-tetramethyl-6-oxo-2-naphthalenyl)-1-propenyl]benzonitril

Zu einer Suspension von 230 ml Dimethylsulfoxid und 7 g 80 %iges Natriumhydrid, das zuvor mit Petrolether vom 20 %igen Paraffin-Anteil befreit worden war, wird innerhalb 1 h eine Lösung von 56 g p-Cyanobenzyl-phosphonsäurediethylester in 115 ml DMSO bei ca. 40C zugetropft. Anschließend wird noch 30 min bei Raumtemperatur gerührt und innerhalb von 10 min 38 g des Ketongemisches 6-Acetyl-1,1,4,4-tetramethyl-3-tetralon und 6-Acetyl-1,1,4,4-tetramethyl-2-tetralon in 50 ml Dimethylsulfoxid und 150 ml Tetrahydrofuran zugetropft. Nach 3 h wird der Ansatz auf Eiswasser gegossen und mit verdünnter Salzsäure angesäuert. Der entstandene Feststoff wird abfiltriert und auf dem Filter nacheinander mit Methanol und Essigsäureethylester gewaschen.

Der Feststoff wird anschließend in 350 ml Aceton und 350 ml Tetrahydrofuran gelöst und diese Lösung langsam mit 350 ml Wasser versetzt. Das entstandene Kristallisat wird abgesaugt, mit Methanol gewaschen und getrocknet. Es verbleiben 25 g eines Gemisches aus 7 Teilen der 7-Oxo-Verbindung und 3 Teilen der Titelverbindung. Die Strukturzuordnung erfolgt über ein NO-Experiment. Aus der Mutterlauge kristallisieren nach eintätigem Stehen, 3,3 g der Titelverbindung, Schmp. 170-171 °C.

b) (E)-4-[2-(5,6,7,8-Tetrahydro-5,5,8,8-tetramethyl-7-oxo-2-naphthalenyl)-1-propenyl] benzonitril

Aus 25 g des vorangehend beschriebenen Gemisches aus 7 Teilen des 7-oxo- und 3 Teilen des 6-Oxobenzonitrils erhält man durch mehrmaliges Umkristallisieren aus Essigester 15,1 g der Titelverbindung, Schmp. 222-223 °C.

Beispiel 5

(E)-4-[2-(5,6,7,8-Tetrahydro-5,5,8,8-tetramethyl-6-oxo-2-naphthalenyl)-1-propenyl]benzoesäure

Analog zu der in Beispiel 2 beschriebenen Arbeitsweise erhält man aus 2,1 g (E)-4-[2-(5,6,7,8-Tetrahydro-5,5,8,8-tetramethyl-6-oxo-2-naphthalenyl)-1-propenyl]benzonitril und 30 ml 10 N Natronlauge in 30 ml Propanol-2 nach 7 h Reaktionszeit 2,5 g der Titelverbindung vom Schmp. 259-250 °C.

Beispiel 6

(E)-4-[2-(5,6,7,8-Tetrahydro-5,5,8,8-tetramethyl-7-hydroxy-2-naphthalenyl)-1-propenyl]benzaldehyd

Zu einer gerührten Suspension von 5,1 g (E)-4-[2-(5,6,7,8-Tetrahydro-5,5,8,8-tetramethyl-7-oxo-2-naphthalenyl)-1-propenyl]benzonitril gibt man 50 ml einer 1,2 molaren Lösung von DIBAL in Toluol. Nach 2 h bei Raumtemperatur versetzt man mit einer gesättigten Weinsäurelösung und extrahiert mehrmals mit Ether. Die vereinigten organischen Phasen werden mit Wasser neutral gewaschen, über Natriumsulfat getrocknet und vom Lösungsmittel befreit. Der verbleibende Rohaustrag wird einer Flash-Chromatographie an Kieselgel unterworfen. Es verbleiben 3,2 g der Titelverbindung in amorpher Gestalt. Das Material kann gegebenenfalls so direkt weiterverarbeitet werden.

Beispiel 7

(E)-4-[2-(5,6,7,8-Tetrahydro-5,5,8,8-tetramethyl-7-hydroxy-2-naphthalenyl)-1-propenyl]benzylalkohol

3,2 g des Aldehyds aus Beispiel 6 wird gemäß Beispiel 3 mit Natriumborhydrid in iso-Propanol reduziert. Man erhält 1,3 g der Titelverbindung, Schmp. 182-183 °C.

Beispiel 8

(E)-4-[2-(5,6,7,8-Tetrahydro-5,5,8,8-tetramethyl-7-hydroxy-2-naphthalenyl)-1propenyl]benzonitril

Zu einem Gemisch aus 6,1 g (30 mmol) Aluminiumisopropylat, 60 ml abs. Isopropanol und 30 ml Toluol werden 2,2 g (6,5 mmol) des Ketonitrils aus Beispiel 4 b) bei Rückflußtemperatur zugegeben. Innerhalb 8 h werden ca. 100 ml Lösungsmittelgemisch abdestilliert und durch die gleiche Menge Isopropanol + Toluol (9 : 1) ersetzt. Dieser Vorgang wird wiederholt, bis kein Aceton mehr im Destillat nachzuweisen ist (Dinitrophenylhydrazinprobe).

Man läßt abkühlen, engt zur Trockne ein und versetzt mit 20 g Eis und 20 ml 2 N HCl. Man verrührt kräftig, saugt den Feststoff ab, wäscht ihn mit 5 ml Methanol nach und erhält nach Trocknen 2,1 g der Titelverbindung, Schmp. 163-165 °C.

Beispiel 9

(E)-4-[2-(5,6,7,8-Tetrahydro-5,5,8,8-tetramethyl-7-hydroxy-2-naphthalenyl)-1-propenyl]benzoesäure

Analog zu der in Beispiel 2 beschriebenen Arbeitsweise erhält man aus 2,1 g (E)-4-[2-(5,6,7,8-Tetrahydro-5,5,8,8-tetramethyl-7-hydroxy-2-naphthalenyl)-1-propenyl] benzonitril (Beispiel 8) und 42 ml 10 N Natronlauge in 42 ml Ethanol nach ca. 5 h Reaktionszeit 1,5 g der Titelverbindung, Schmp. 250-252 °C.

Beispiel 10

(E)-4-[2-(5,6,7,8-Tetrahydro-5,5,8,8-tetramethyl-7-oxo-2-naphthalenyl)-1-propenyl]benzoesäureazid

Zu einer Suspension von 12,7 g (35 mmol) (E)-4-[2-(5,6,7,8-Tetrahydro-5,5,8,8-tetramethyl-7-oxo-2-naphthalenyl)-1-propenyl]benzoesäure (Beispiel 2) in 60 ml Aceton und 20 ml Wasser wird bei 0-5 °C eine Lösung von 6 ml Triethylamin in 30 ml Aceton zulaufen gelassen. Bei 0 °C tropft man 4,5 ml (5,2 g, 55 mmol) Chlorameisensäureethylester zu, rührt 10 min. nach, und tropft dann eine Lösung von 3,5 g (55 mmol) Natriumazid in 7,5 ml Wasser zu. Man läßt 2 h bei 0 °C nachrühren, saugt den feinkristallinen Feststoff ab, wäscht mit wenig Wasser und Ethanol nach und erhält nach Trocknen 10,8 g der Titelverbindung, die ohne weitere Reinigung weiterverarbeitet wird (s. Beispiel 11).

Beispiel 11

(E)-4-[2-(5,6,7,8-Tetrahydro-5,5,8,8-tetramethyl-7-oxo-2-naphthalenyl)-1-propenyl]benz(2-hydroxyethyl)amid

Zu einer Lösung von 1,9 g (5 mmol) des Säureazids aus Beispiel 10 in 100 ml THF werden 10 ml Ethanolamin zugegeben. Man verrührt kurz und läßt 20 min. bei Raumtemperatur stehen. Die klare Lösung wird auf 400 ml Wasser gegossen, angesäuert und der entstandene Niederschlag abgesaugt. Man wäscht mit 5 ml Methanol nach und erhält nach Trocknen und Umkristallisieren aus Essigester/Toluol (3 : 1) 1,1 g der Titelverbindung, Schmp. 201-203 °C.

Beispiel 12

(E)-4-[2-(5,6,7,8-Tetrahydro-5,5,8,8-tetramethyl-7-oxo-2-naphthalenyl)-1-propenyl]benz(n-butyl)amid

Zu einem Gemisch aus 3,6 g (10 mmol) (E)-4-[2-(5,6,7,8-Tetra-5,5,8,8-tetramethyl-7-oxo-2-naphthalenyl)-1-propenyl]benzoesäure (Beispiel 2), 1,8 ml (12,5 mmol) Triethylamin und 40 ml Aceton werden bei 0 °C 1,4 ml (1,6 g, 15 mmol) Chlorameisensäureethylester in 10 ml Aceton zugetropft. Man läßt 30 min. bei 0 °C nachrühren und tropft dann bei 0 °C eine Lösung von 1,5 g (20 mmol) n-Butylamin in 20 ml Aceton zu. Man läßt auf Raumtemperatur kommen, verdünnt mit 30 ml Aceton, gibt nach 2 h nochmals 3 g (40 mmol) n-Butylamin zu und läßt über Nacht nachrühren.

Man gibt auf 400 ml Wasser, säuert mit 2 N HCl an und saugt den entstandenen Feststoff ab, der mit Wasser und Methanol gewaschen und getrocknet wird. Das so erhaltene Rohprodukt (3,6 g) wird aus 200 ml Methanol umkristallisiert und schließlich einer Blitzchromatographie (200 g Si 60 ; n-Heptan mit steigendem Anteil Essigester) unterworfen. Man erhält so 1 g der Titelverbindung, Schmp. 172-174 °C.

**Patentansprüche** (für die Vertragsstaaten : BE, CH, DE, FR, GB, IT, LI, NL, SE)

1. Tetralin-Derivate der Formel I

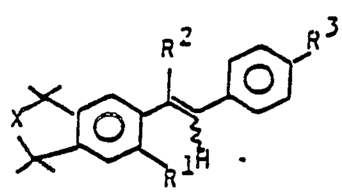

(I)

in der

$R^1$ ein Wasserstoffatom, eine $C_{1-4}$-Alkyl- oder $C_{1-4}$-Alkoxygruppe oder ein Halogenatom,

$R^2$ ein Wasserstoffatom oder eine $C_{1-6}$-Alkyl- oder $C_{3-6}$-Cycloalkylgruppe,

X eine $—CH_2—CO—$, $—CH_2—CHOH—$, $—CH_2—C(R^4)OH—$, $—CH_2—C(OCOR^4)H—$, $—CH_2—C(R^4)(OCHOR^5)—$, $—CHOH—CHOH—$, $—CH(OCOR^4)—CH(OCOR^4)—$, $—CH_2—CH(OR^4)—$ oder

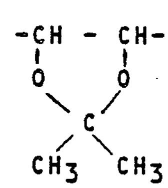

(I)

-Gruppe (mit $R^4$ und $R^5$ in der Bedeutung von $C_{1-4}$-Alkyl) und

$R^3$ eine Nitril- oder $C_{2-10}$-Ketalgruppe oder eine —CH($R^7$)($R^8$)— oder —CO—$R^9$, worin $R^7$ ein Wasserstoffatom oder eine $C_{1-3}$-Alkylgruppe, $R^8$ ein Wasserstoffatom, eine $C_{1-3}$-Alkylgruppe oder einen Rest —$OR^{10}$ oder —$NR^{11}R^{12}$ (mit $R^{10}$ in der Bedeutung von Wasserstoff, $C_{1-4}$-Alkyl, $C_{2-20}$-Alkanoyl oder gegebenenfalls substituiertem Benzoyl und $R^{11}$ und $R^{12}$ in der Bedeutung von Wasserstoff, $C_{1-4}$-Alkyl, $C_{2-20}$-Alkanoyl, gegebenenfalls substituiertem Benzoyl oder $R^{11}$ und $R^{12}$ zusammen mit dem Stickstoffatom, an das sie gebunden sind, in der Bedeutung eines heterocyclischen Rests), und $R^9$ ein Wasserstoff- oder Halogenatom, eine $C_{1-4}$-Alkylgruppe, den Azido-, Imidazol- oder Triazolrest oder die Gruppen —$OR^{13}$ odser —$NR^{14}R^{15}$ bedeutet (mit $R^{13}$ in der Bedeutung von Wasserstoff, $C_{1-8}$-Alkyl, mit einer oder mehreren Hydroxygruppen oder einer $C_{1-4}$-Alkoxygruppe substituiertes $C_{2-6}$-Alkyl, gegebenenfalls substituiertes Aryl, gegebenenfalls im Arylteil substituiertes Aralkyl und $R^{14}$ und $R^{15}$ in der Bedeutung von Wasserstoff, $C_{1-6}$-Alkyl, mit einer oder mehreren Hydroxygruppen substituiertes $C_{2-6}$-Alkyl, einer Aryl- oder Tetrazoylgruppe oder $R^{14}$ und $R^{15}$ zusammen mit dem Stickstoffatom, an das sie gebunden sind, in der Bedeutung eines heterocyclischen Ringsystems mit 3-6 Ringatomen bedeuten, sowie gegebenenfalls deren physiologisch verträglichen Salze.

2. Tetralin-Derivat der Formel I gemäß Anspruch 1, dadurch gekennzeichnet, daß sie in der E-Form vorliegen.

3. Tetralin-Derivate der Formel I gemäß Anspruch 1 oder 2, dadurch gekennzeichnet, daß X die Gruppen —$CH_2$—CO—, —$CH_2$—CHOH— oder —CHOH—CHOH, $R^1$ Wasserstoff, Methyl, Methoxy oder Fluor, $R^2$ Wasserstoff oder Methyl und $R^3$ die Gruppen —COOH, —COO—$C_{1-4}$-alkyl, —CHO, —$CH_2OH$ oder $CH_2$—O—CO—$CH_3$ bedeuten.

4. (E)-4-[2-(5,6,7,8-Tetrahydro-5,5,8,8-tetramethyl-7-hydroxy-2-naphthalenyl)-1-propenyl]benzylalkohol.

5. (E)-4-[2-(5,6,7,8-Tetrahydro-5,5,8,8-tetramethyl-6-oxo-2-naphthalenyl)-1-propenyl]benzoesäure.

6. (E)-4-[2-(5,6,7,8-Tetrahydro-5,5,8,8-tetramethyl-7-hydroxy-2-naphthalenyl)-1-propenyl]benzoesäure.

7. (E)-4-[-(5,8-Dihydro-5,5,8,8-tetramethyl-2-naphthalenyl)-1-propenyl]benzosäure.

8. Tetralin-Derivate der Formel I gemäß Anspruch 1 zur Verwendung bei der Bekämpfung von Krankheiten.

**Patentansprüche** (für den Vertragsstaat AT)

1. Verfahren zur Herstellung der Tetralin-Derivate der Formel I

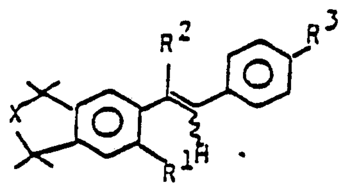

(I)

in der

$R^1$ ein Wasserstoffatom, eine $C_{1-4}$-Alkyl- oder $C_{1-4}$-Alkoxygruppe oder ein Halogenatom,
$R^2$ ein Wasserstoffatom oder eine $C_{1-6}$-Alkyl- oder $C_{3-6}$-Cycloalkylgruppe,
X eine —$CH_2$—CO—, —$CH_2$—CHOH—, —$CH_2$—C($R^4$)OH—, —$CH_2$—C($OCOR^4$)H—, —$CH_2$—C($R^4$) ($OCHOR^5$)—, —CHOH—CHOH—, —CH($OCOR^4$)—CH($OCOR^4$)—, —$CH_2$—CH($OR^4$)— oder

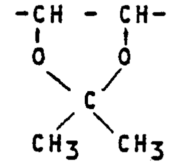

-Gruppe (mit $R^4$ und $R^5$ in der Bedeutung von $C_{1-4}$-Alkyl) und

$R^3$ eine Nitril- oder $C_{2-10}$-Ketalgruppe oder eine —CH($R^7$) ($R^8$)— oder —CO—$R^9$, worin $R^7$ ein Wasserstoffatom oder eine $C_{1-3}$-Alkylgruppe, $R^8$ ein Wasserstoffatom, eine $C_{1-3}$-Alkylgruppe oder einen Rest —$OR^{10}$ oder —$NR^{11}R^{12}$ (mit $R^{10}$ in der Bedeutung von Wasserstoff, $C_{1-4}$-Alkyl, $C_{2-20}$-Alkanoyl oder gegebenenfalls substituiertem Benzoyl und $R^{11}$ und $R^{12}$ in der Bedeutung von Wasserstoff, $C_{1-4}$-Alkyl, $C_{2-20}$-Alkanoyl, gegebenenfalls substituiertem Benzoyl oder $R^{11}$ und $R^{12}$ zusammen mit dem Stickstoffatom, an das sie gebunden sind, in der Bedeutung eines heterocyclischen Rests), und $R^9$ ein Wasserstoff- oder Halogenatom, eine $C_{1-4}$-Alkylgruppe, den Azido-, Imidazol- oder Triazolrest oder die Gruppen —$OR^{13}$ oder —$NR^{14}R^{15}$ bedeutet (mit $R^{13}$ in der Bedeutung von Wasserstoff, $C_{1-8}$-Alkyl, mit einer oder mehreren Hydroxygruppen oder einer $C_{1-4}$-Alkoxygruppe substituiertes $C_{2-6}$-Alkyl, gegebe-

nenfalls substituiertes Aryl, gegebenenfalls im Arylteil substituiertes Aralkyl und $R^{14}$ und $R^{15}$ in der Bedeutung von Wasserstoff, $C_{1-6}$-Alkyl, mit einer oder mehreren Hydroxygruppen substituiertes $C_{2-6}$-Alkyl, einer Aryl- oder Tetrazoylgruppe oder $R^{14}$ und $R^{15}$ zusammen mit dem Stickstoffatom, an das sie gebunden sind, in der Bedeutung eines heterocyclischen Ringsystems mit 3-6 Ringatomen) bedeuten, sowie gegebenenfalls deren physiologisch verträglichen Salze, dadurch gekennzeichnet, daß man eine Carbonylverbindung der Formel (II)

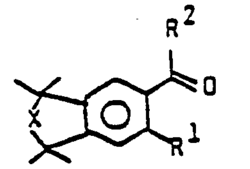

(II)

in der X, $R^1$ und $R^2$ die oben angegebenen Bedeutungen haben mit einer Phosphorverbindung der Formel (III)

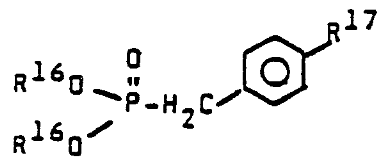

(III)

in der $R^{16}$ eine $C_{1-3}$-Alkylgruppe und $R^{17}$ eine $C_{1-2}$-Alkylgruppe, eine Carbo-$C_{1-8}$-alkoxygruppe oder eine Nitrilgruppe bedeutet oder für eine ($C_{1-4}$-Alkoxy)methylgruppe nach Wittig-Horner umsetzt und die so erhaltenen Verbindungen gegebenenfalls

A) — falls $R^3$ den Rest $COR^9$ bedeutet — einen Ester der Formel I (mit $R^3$ in der Bedeutung einer Carbo-$C_{1-8}$-alkoxygruppe) verseift und die so erhaltene Säure gegebenenfalls verestert, amidiert, reduziert oder —falls $R^3$ die Nitrilgruppe bedeutet — zur Carbonsäure oder Säureamid verseift oder zum Aldehyd reduziert und gegebenenfalls

a) so erhaltene Alkohole verestert, verethert oder zu den entsprechenden Aldehyden oxidiert oder

b) so erhaltene Amine in ihre Amide überführt und die erhaltenen Verbindungen gegebenenfalls in ihre physiologisch verträgliche Salze überführt.

2. Verfahren gemäß Anspruch 1, dadurch gekennzeichnet, daß man die (E)-Verbindungen herstellt.

3. Verfahren gemäß Anspruch 1 oder 2, dadurch gekennzeichnet, daß man Verbindungen herstellt, in denen X die Gruppen X die Gruppen $-CH_2-CO-$, $-CH_2-CHOH-$ oder $-CHOH-CHOH$, $R^1$ Wasserstoff, Methyl, Methoxy oder Fluor, $R^2$ Wasserstoff oder Methyl und $R^3$ die Gruppen $-COOH$, $-COO-C_{1-4}$-alkyl, $-CHO$, $-CH_2OH$ oder $CH_2-O-CO-CH_3$ bedeuten.

4. Verfahren gemäß Anspruch 1, dadurch gekennzeichnet, daß man (E)-4-[2-(5,6,7,8-Tetrahydro-5,5,8,8-tetramethyl-7-hydroxy-2-naphthal enyl)1-propenyl]benzylalkohol herstellt.

5. Verfahren gemäß Anspruch 1, dadurch gekennzeichnet, daß man (E)-4-[2-(5,6,7,8-Tetrahydro-5,5,8,8-tetramethyl-6-oxo-2-naphthalenyl)-1-propenyl]benzoesäure herstellt.

6. Verfahren gemäß Anspruch 1, dadurch gekennzeichnet, daß man (E)-4-[2-(5,6,7,8-Tetrahydro-5,5,8,8-tetramethyl-7-hydroxy-2-naphthalenyl)-1-propenyl]benzoesäure herstellt.

7. Verfahren gemäß Anspruch 1, dadurch gekennzeichnet, daß man (E)-4-[2-(5,8-Dihydro-5,5,8,8-tetramethyl-2-naphthalenyl)-1-propenyl]benzosäure herstellt.

**Claims** (for the Contracting States : BE, CH, DE, FR, GB, IT, LI, NL, SE)

1. A tetralin derivative of the formula I

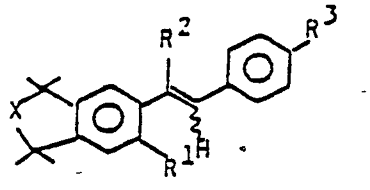

(I)

where $R^1$ is hydrogen, $C_1$-$C_4$-alkyl, $C_1$-$C_4$-alkoxy or halogen, $R^2$ is hydrogen, $C_1$-$C_6$-alkyl or $C_3$-$C_6$-cycloalkyl, X is $-CH_2-CO-$, $-CH_2-CHOH$, $-CH_2-C(R^4)OH-$, $-CH_2-C(OCOR^4)H-$, $-CH_2-C(R^4)(OCHOR^5)-$, $-CHOH-CHOH-$, $-CH(OCOR^4)-CH(OCOR^4)-$, $-CH_2-CH(OR^4)-$ or

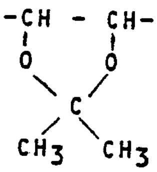

(where $R^4$ and $R^5$ are each $C_1$-$C_4$-alkyl) and $R^3$ is nitrile, $C_2$-$C_{10}$-ketal or —CH($R^7$) ($R^8$)— or —CO—$R^9$, where $R^7$ is hydrogen or $C_1$-$C_3$-alkyl, $R^8$ is hydrogen, $C_1$-$C_3$-alkyl or —O$R^{10}$ or —N$R^{11}R^{12}$ (where $R^{10}$, $R^{11}$ and $R^{12}$ are each hydrogen, $C_1$-$C_4$-alkyl, $C_2$-$C_{20}$-alkanoyl or unsubstituted or substituted benzoyl, or $R^{11}$ and $R^{12}$, together with the nitrogen atom to which they are bonded, form a heterocyclic radical) and $R^9$ is hydrogen, halogen, $C_1$-$C_4$-alkyl, azido, imidazolyl, triazolyl or —O$R^{13}$ or —N$R^{14}R^{15}$ (where $R^{13}$ is hydrogen or $C_1$-$C_8$-alkyl or is $C_2$-$C_6$-alkyl which is substituted by one or more hydroxyl groups or a $C_1$-$C_4$-alkoxy group, or is unsubstituted or substituted aryl or aralkyl which is unsubstituted or substituted in the aryl moiety, and $R^{14}$ and $R^{15}$ are each hydrogen or $C_1$-$C_6$-alkyl or are each $C_2$-$C_6$-alkyl which is substituted by one or more hydroxyl groups or are each aryl or tetrazolyl, or $R^{14}$ and $R^{15}$, together with the nitrogen atom to which they are bonded, form a heterocyclic ring system possessing 3 to 6 ring atoms), and, where relevant, its physiologically tolerated salts.

2. A tetralin derivative of the formula I as claimed in claim 1, which is present in the E form.

3. A tetralin derivative of the formula I as claimed in claim 1 or 2, wherein X is —$CH_2$—CO—, —$CH_2$—CHOH— or —CHOH—CHOH, $R^1$ is hydrogen, methyl, methoxy or fluorine, $R^2$ is hydrogen or methyl, and $R^3$ is —COOH, —COO—$C_1$-$C_4$-alkyl, —CHO, —$CH_2$OH or —$CH_2$—O—CO—$CH_3$.

4. (E)-4-[2-(5,6,7,8-Tetrahydro-5,5,8,8-tetramethyl-7-hydroxynaphth-2-yl)-1-propenyl]-benzyl alcohol.

5. (E)-4-[2-(5,6,7,8-Tetrahydro-5,5,8,8-tetramethyl-6-oxonaphth-2-yl)-1-propenyl]-benzoic acid.

6. (E)-4-[2-(5,6,7,8-Tetrahydro-5,5,8,8-tetramethyl-7-hydroxynaphth-2-yl)-1-propenyl]-benzoic acid.

7. (E)-4-[2-(5,8-Dihydro-5,5,8,8-tetramethyl-naphth-2-yl)-1-propenyl]-benzoic acid.

8. A tetralin derivative of the formula I as claimed in claim 1 for use in the treatment of disorders.

**Claims** (for the Contracting State AT)

1. A process for the preparation of a tetralin derivative of the formula I

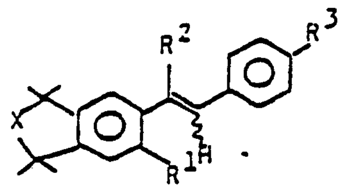

(I)

where $R^1$ is hydrogen, $C_1$-$C_4$-alkyl, $C_1$-$C_4$-alkoxy or halogen, $R^2$ is hydrogen, $C_1$-$C_6$-alkyl or $C_3$-$C_6$-cycloalkyl, X is —$CH_2$—CO—, —$CH_2$—CHOH, —$CH_2$—C($R^4$)OH—, —$CH_2$—C(OCO$R^4$)H—, —$CH_2$—C($R^4$) (OCHO$R^5$)—, —CHOH—CHOH—, —CH(OCO$R^4$)—CH(OCO$R^4$)—, —$CH_2$—CH(O$R^4$)— or

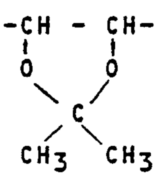

(where $R^4$ and $R^5$ are each $C_1$-$C_4$-alkyl) and $R^3$ is nitrile, $C_2$-$C_{10}$-ketal or —CH($R^7$) ($R^8$)— or —CO—$R^9$, where $R^7$ is hydrogen or $C_1$-$C_3$-alkyl, $R^8$ is hydrogen, $C_1$-$C_3$-alkyl or —O$R^{10}$ or —N$R^{11}R^{12}$ (where $R^{10}$, $R^{11}$ and $R^{12}$ are each hydrogen, $C_1$-$C_4$-alkyl, $C_2$-$C_{20}$-alkanoyl or unsubstituted or substituted benzoyl, or $R^{11}$ and $R^{12}$, together with the nitrogen atom to which they are bonded, form a heterocyclic radical) and $R^9$ is hydrogen, halogen, $C_1$-$C_4$-alkyl, azido, imidazolyl, triazolyl or —O$R^{13}$ or —N$R^{14}R^{15}$ (where $R^{13}$ is hydrogen or $C_1$-$C_8$-alkyl or is $C_2$-$C_6$-alkyl which is substituted by one or more hydroxyl groups or a $C_1$-$C_4$-alkoxy group, or is unsubstituted or substituted aryl or aralkyl which is unsubstituted or substituted in the aryl moiety, and $R^{14}$ and $R^{15}$ are each hydrogen or $C_1$-$C_6$-alkyl or are each $C_2$-$C_6$-alkyl which is substituted by one or more hydroxyl groups or are each aryl or tetrazolyl, or $R^{14}$ and $R^{15}$, together with the nitrogen atom to which they are bonded, form a heterocyclic ring system possessing 3 to 6 ring atoms), and, where relevant, its physiologically tolerated salts, wherein a carbonyl compound of the formula (II)

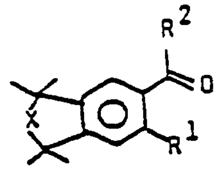

(II)

where X, $R^1$ and $R^2$ have the meanings stated above is subjected to a Wittig-Horner reaction with a phosphorus compound of the formula (III)

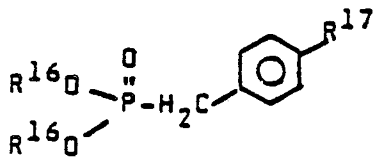

(III)

where $R^{16}$ is $C_1$-$C_3$-alkyl and $R^{17}$ is $C_1$-$C_2$-alkyl, carbo-$C_1$-$C_8$-alkoxy, nitrile or ($C_1$-$C_4$-alkoxy)methyl, and, if desired, the resulting compound is

A) where $R^3$ is $COR^9$ — hydrolyzed with an ester of the formula I (where $R^3$ is carbo-$C_1$-$C_8$-alkoxy) and the resulting acid is, if desired, esterified, amidated, reduced or — where $R^3$ is nitrile — hydrolyzed to give carboxylic acid or an acid amide or reduced to give the aldehyde and, if desired,

a) the alcohol thus obtained is esterified, etherified or oxidized to the corresponding aldehyde or

b) the resulting amine is converted to its amide and the resulting compound is, if desired, converted into its physiologically tolerated salts.

2. A process as claimed in claim 1, wherein the (E) compound is prepared.

3. A process as claimed in claim 1 or 2, wherein compounds are prepared in which X is $—CH_2—CO—$, $—CH_2—CHOH—$ or $—CHOH—CHOH$, $R^1$ is hydrogen, methyl, methoxy or fluorine, $R^2$ is hydrogen or methyl, and $R^3$ is $—COOH$, $—COO—C_1$-$C_4$-alkyl, $—CHO$, $—CH_2OH$ or $—CH_2—O—CO—CH_3$.

4. A process as claimed in claim 1, wherein (E)-4-[2-(5,6,7,8-tetrahydro-5,5,8,8-tetramethyl-7-hydroxy-naphth-2-yl)-1-propenyl]-benzyl alcohol is prepared.

5. A process as claimed in claim 1, wherein (E)-4-[2-(5,6,7,8-tetrahydro-5,5,8,8-tetramethyl-6-oxo-naphth-2-yl)-1-propenyl]-benzoic acid is prepared.

6. A process as claimed in claim 1, wherein (E)-4-[2-(5,6,7,8-tetrahydro-5,5,8,8-tetramethyl-7-hydroxy-naphth-2-yl)-1-propenyl]-benzoic acid is prepared.

7. A process as claimed in claim 1, wherein (E)-4-[2-(5,8-dihydro-5,5,8,8-tetramethyl-naphth-2-yl)-1-propenyl]-benzoic acid is prepared.

**Revendications** (pour les Etats contractants : BE, CH, DE, FR, GB, IT, LI, LU, NL, SE)

1. Dérivés de tétraline de formule I

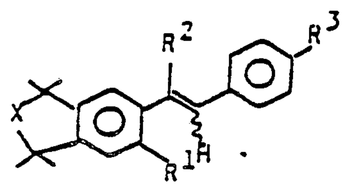

(I)

dans laquelle

$R^1$ représente un atome d'hydrogène, un groupe alkyle en $C_{1-4}$ ou alcoxy en $C_{1-4}$, ou un atome d'halogène,

$R^2$, un atome d'hydrogène ou un alkyle en $C_{1-6}$ ou cycloalkyle en $C_{3-6}$,

X, un groupe $—CH_2—CO—$, $—CH_2—CHOH—$, $—CH_2—C(R^4)OH—$, $—CH_2—C(OCOR^4)H—$, $—CH_2—C(R^4)(OCHOR^5)—$, $—CHOH—CHOH—$, $—CH(OCOR^4)—CH(OCOR^4)—$, $—CH_2—CH(OR^4)—$ ou

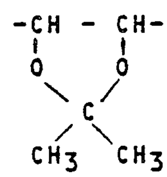

(avec $R^4$ et $R^5$ signifiant alkyle en $C_{1-4}$) et $R^3$ représente un groupe nitrile ou cétal en $C_{2-10}$ ou un groupe —CH ($R^7$) ($R^8$)— ou —CO—$R^9$, où $R^7$ représente un atome d'hydrogène ou un groupe alkyle en $C_{1-3}$, $R^8$ un atome d'hydrogène, un groupe alkyle en $C_{1-3}$ ou un reste —$OR^{10}$ ou —$NR^{11}R^{12}$ (avec $R^{10}$ signifiant hydrogène, alkyle en $C_{1-4}$, alcanoyle en $C_{2-20}$ ou benzoyle éventuellement substitué et $R^{11}$ et $R^{12}$ signifiant hydrogène, alkyle en $C_{1-4}$, alcanoyle en $C_{2-20}$ ou benzoyle éventuellement substitué et $R^{11}$ et $R^{12}$ signifiant hydrogène, alkyle en $C_{1-4}$, alcanoyle en $C_{2-20}$ benzoyle éventuellement substitué, ou $R^{11}$ et $R^{12}$ avec l'atome d'azote, auquel ils sont liés, signifiant un reste hétérocyclique) et $R^9$ un atome d'hydrogène ou d'halogène, un groupe alkyle en $C_{1-4}$, le reste azido, imidazole ou triazole ou les groupes —$OR^{13}$ ou —$NR^{14}R^{15}$ (avec $R^{13}$ signifiant hydrogène, alkyle $C_{1-8}$, alkyle en $C_{2-6}$ substitué avec un ou plusieurs groupes hydroxy ou un groupe alcoxy en $C_{1-4}$, aryle éventuellement substitué, aralkyle éventuellement substitué dans la partie aryle et $R^{14}$ et $R^{15}$ signifiant hydrogène, alkyle en $C_{1-6}$, alkyle en $C_{2-6}$ substitué avec un ou plusieurs groupes hydroxy, un groupe aryle ou tétrazoyle ou $R^{14}$ et $R^{15}$, avec l'atome d'azote auquel ils sont liés, signifiant un système hétérocyclique à 3-6 atomes du cycle ainsi qu'éventuellement leurs sels acceptables physiologiquement.

2. Dérivés de tétraline de formule I selon la revendication 1, caractérisés par le fait qu'ils se présentent sous forme E.

3. Dérivés de tétraline de formule I selon la revendication 1 ou 2, caractérisés par le fait que
X représente les groupes —$CH_2$—CO—, —$CH_2$—CHOH— ou —CHOH—CHOH,
$R^1$ hydrogène, méthyle, méthoxy ou fluor,
$R^2$ hydrogène ou méthyle et
$R^3$ les groupes —COOH, —COO-alkyle en $C_{1-4}$, —CHO, —$CH_2OH$ ou $CH_2$—O—CO—$CH_3$.

4. Alcool (E)-4-[2-(5,6,7,8-tétraméthyl-7-hydroxy-2-naphtalényl-1-propényl]benzylique.

5. Acide (E)-4-[2-(5,6,7,8-tétrahydro-5,5,8,8-tétraméthyl-6-oxo-2-naphtalényl)-1-propényl]benzoïque.

6. Acide (E)-4-[2-(5,6,7,8-tétrahydro-5,5,8,8-tétraméthyl-7-hydroxy-2-naphtalényl)-1-propényl]benzoïque.

7. Acide (E)-4-[2-(5,8-dihydro-5,5,8,8-tétraméthyl-2-naphtalényl)-1-propényl]benzoïque.

8. Dérivés de tétraline de formule I selon la revendication 1 pour utilisation dans la lutte contre des maladies.

**Revendications** (pour les Etats contractants : BE, CH, DE, FR, GB, IT, LI, LU, NL, SE)

1. Procédé de préparation de dérivés de tétraline de formule I

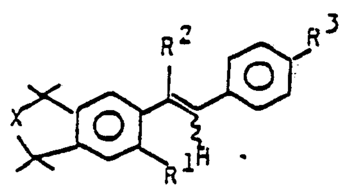

(I)

dans laquelle

$R^1$ représente un atome d'hydrogène, un groupe alkyle en $C_{1-4}$ ou alcoxy en $C_{1-4}$, ou un atome d'halogène,

$R^2$, un atome d'hydrogène ou un alkyle en $C_{1-6}$ ou cycloalkyle en $C_{3-6}$,

X, un groupe —$CH_2$—CO—, —$CH_2$—CHOH—, —$CH_2$—C($R^4$)OH—, —$CH_2$—C($OCOR^4$)H—, —$CH_2$—C($R^4$) ($OCHOR^5$)—, —CHOH—CHOH—, —CH($OCOR^4$)—CH($OCOR^4$)—, —$CH_2$—CH($OR^4$)— ou

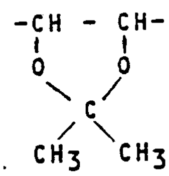

(avec $R^4$ et $R^5$ signifiant alkyle en $C_{1-4}$) et

$R^3$ représente un groupe nitrile ou cétal en $C_{2-10}$ ou un groupe —CH ($R^7$) ($R^8$)— ou —CO—$R^9$, où $R^7$ représente un atome d'hydrogène ou un groupe alkyle en $C_{1-3}$, $R^8$ un atome d'hydrogène, un groupe alkyle en $C_{1-3}$ ou un reste —$OR^{10}$ ou —$NR^{11}R^{12}$ (avec $R^{10}$ signifiant hydrogène, alkyle en $C_{1-4}$, alcanoyle en $C_{2-20}$ ou benzoyle éventuellement substitué et $R^{11}$ et $R^{12}$ signifiant hydrogène, alkyle en $C_{1-4}$, alcanoyle en $C_{2-20}$ ou benzoyle éventuellement substitué et $R^{11}$ et $R^{12}$ signifiant hydrogène, alkyle en $C_{1-4}$, alcanoyle en $C_{2-20}$ benzoyle éventuellement substitué, ou $R^{11}$ et $R^{12}$ avec l'atome d'azote, auquel ils sont liés, signifiant un reste hétérocyclique) et $R^9$ un atome d'hydrogène ou d'halogène, un groupe alkyle en $C_{1-4}$, le reste azido, imidazole ou triazole ou les groupes —$OR^{13}$ ou —$NR^{14}R^{15}$ (avec

$R^{13}$ signifiant hydrogène, alkyle $C_{1-8}$, alkyle en $C_{2-6}$ substitué avec un ou plusieurs groupes hydroxy ou un groupe alcoxy en $C_{1-4}$, aryle éventuellement substitué, aralkyle éventuellement substitué dans la partie aryle et $R^{14}$ et $R^{15}$ signifiant hydrogène, alkyle en $C_{1-6}$, alkyle en $C_{2-6}$ substitué avec un ou plusieurs groupes hydroxy, un groupe aryle ou tétrazoyle ou $R^{14}$ et $R^{15}$, avec l'atome d'azote auquel ils sont liés, signifiant un système hétérocyclique à 3-6 atomes du cycle ainsi qu'éventuellement leurs sels acceptables physiologiquement caractérisé par le fait qu'on fait réagir selon Wittig-Horner un composé carbonyle de formule II

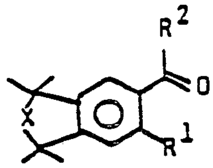

(II)

dans laquelle

X, $R^1$ et $R^2$ ont les significations sus-indiquées, avec un composé de phosphore de formule III

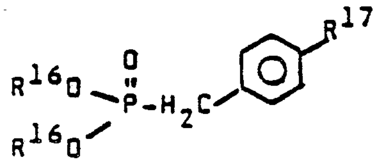

(III)

dans laquelle $R^{16}$ représente un groupe alkyle en $C_{1-3}$ et $R^{17}$ un groupe alkyle en $C_{1-2}$, un groupe carbo-alcoxy en $C_{1-8}$ ou un groupe nitrile ou est mis pour un groupe alcoxy en $C_{1-4}$-méthyle et les composés ainsi obtenus sont éventuellement

A) dans le cas où $R^3$ représente le reste $COR^9$ — un ester de formule I (avec $R^3$ représentant un groupe carbo-alcoxy en $C_{1-8}$) saponifiés et l'acide ainsi obtenu éventuellement estérifié, amidé, réduit ou — dans le cas ou $R^3$ représente le groupe nitrile-saponifié en acide carboxylique ou amide d'acide ou réduit en aldéhyde et éventuellement

a) les alcools ainsi obtenus sont estérifiés, éthérifiés ou oxydés en les aldéhydes correspondants, ou

b) les amines ainsi obtenues sont transformées en leurs amides et les composés obtenus sont transformés éventuellement en leurs sels acceptables physiologiquement.

2. Procédé selon la revendication 1, caractérisé par le fait que l'on prépare les composés (E).

3. Procédé selon la revendication 1 ou 2, caractérisé par le fait que l'on prépare des composés dans lesquels

X représente les groupes $—CH_2—CO—$, $—CH_2—CHOH—$ ou $—CHOH—CHOH$,

$R^1$ hydrogène, méthyle, méthoxy ou fluor,

$R^2$ hydrogène ou méthyle et

$R^3$ les groupes $—COOH$, $—COO$-alkyle en $C_{1-4}$, $—CH_2OH$ ou $CH_2—O—CO—CH_3$.

4. Procédé selon la revendication 1, caractérisé par le fait que l'on prépare de l'alcool (E)-4-[2-(5,6,7,8-tétraméthyl-7-hydroxy-2-naphtalényl-1-propényl]benzylique.

5. Procédé selon la revendication 1, caractérisé par le fait que l'on prépare de l'acide (E)-4-[2-(5,6,7,8-tétrahydro-5,5,8,8-tétraméthyl-6-oxo-2-naphtalényl)-1-propényl]benzoïque.

6. Procédé selon la revendication 1, caractérisé par le fait que l'on prépare de l'acide (E)-4-[2-(5,6,7,8-tétrahydro-5,5,8,8-tétraméthyl-7-hydroxy-2-naphtalényl)-1-propényl]benzoïque.

7. Procédé selon la revendication 1, caractérisé par le fait que l'on prépare de l'acide (E)-4-[2-(5,8-dihydro-5,5,8,8-tétraméthyl-2-naphtalényl)-1-propényl]benzoïque.